# EUROPEAN PATENT APPLICATION

(11) **EP 1 854 781 A1**
(43) Date of publication of application: **14.11.2007**
(21) Application number: 05822458.5
(22) Date of filing: 28.12.2005
(51) Int. Cl.: C07C 233/25, A61K 51/00, C07B 59/00

(54) **RADIOACTIVE HALOGEN-LABELED PHENYLOXYANILINE DERIVATIVES**

(30) Priority: 28.02.2005 JP 2005052527
(71) Applicant: TAISHO PHARMACEUTICAL CO., LTD, Tokyo 170-8633 (JP); National Institute of Radiological Sciences, Chiba-shi, Chiba-ken 263-8555 (JP)
(72) Inventor: Suzuki, K., Nat. Inst. of Radiological Sciences, Chiba-shi, Chiba, 2638555 (JP); Suhara, T., Nat. Inst. of Radiological Sciences, Chiba-shi, Chiba, 2638555 (JP); Christar, H., Nat. Inst. of Radioligal Sciences, Chiba-shi, Chiba, 2638555 (JP); Zhang, M.-R., Nat. Inst. of Radiological Sciences, Chiba-shi, Chiba, 2638555 (JP); Nakazato, Atsuro, Taisho Pharmaceutical Co., Ltd., Tokyo 1708633 (JP)
(74) Representative: HOFFMANN EITLE
(86) International application number: PCT/JP2005/024075
(87) International publication number: WO 2006/092902

(57) **Abstract**

A radioactive halogen-labeled phenyoxyaniline derivative represented by the following formula: wherein R¹ represents a group such as an alkyl group; X¹, X², X³ and X⁴ represent each a hydrogen atom, an alkyl group, an alkoxy group, an alkoxy group carrying ¹¹C introduced thereinto or a radioactive halogen atom, provided that at least one of X¹, X², X³ and X⁴ represents an alkoxy group carrying ¹¹C introduced thereinto or a radioactive halogen atom; which is a compound useful as a PBR ligand having a high affinity and a high selectivity. In in vitro measurement of PBR, a PBR ligand having a high affinity and a high selectivity is labeled with a radioactive halogen nuclear species so as to enable the measurement of PBR in vivo with the use of means including not only PET but also SPECT. Thus, a compound which is useful in early diagnosing, preventing and treating diseases such as Alzheimer type dementia can be obtained.

## Description

### TECHNICAL FIELD

The present invention relates to radioactive halogen-labeled phenyloxyaniline derivatives having high affinity to peripheral benzodiazepine receptors.

### BACKGROUND ART

Benzodiazepine receptors (BZ) are classified into central and peripheral receptors. The peripheral benzodiazepine receptor (PBR) was observed at peripherals at first, but the presence thereof was also recognized in the central nervous system. Furthermore, it has been revealed that the density of PBR in the central nervous system is high, almost as high as or higher than that of the central benzodiazepine receptor (CBR) in the same area. Studies up to now have reported that PBR is associated with diseases such as Alzheimer's disease, front-temporal dementia, diffuse Lewy corpuscle disease, vascular lesion, Parkinson's disease-related disease, corticobasilar degeneration, Parkinson's disease, Huntington's chorea, multiple system atrophy, multiple sclerosis, epilepsy, meningitis, encephalitis, peripheral nerve injury, larynx cancer, breast cancer, ovarian tumor, liver cancer, large bowel cancer, stomach cancer, adrenal gland tumor, glioma, glioblastoma, fibroblastoma, neurosarcoma, lung cancer, uterine cancer, lymphoma, prostate cancer, melanoma, testicular tumors, astrocytoma, ectopic hormone-producing tumor.

In imaging the intracerebral PBR distribution of a living human brain with positron emission tomograph (PET), a conventional PBR ligand, ¹¹C-labeled N-methyl-N-(1-methylpropyl)-1-(2-chlorophenyl)-isoquinoline-3-carboxamide (hereinafter PK 11195) has been used to diagnose cerebral glioma and Alzheimer's disease. Since this compound has extremely low accumulation in the brain and has problems in quantitative analysis, however, development of PBR ligand which gives a high signal has been desired. Under the circumstances, it has now become clear that N-(2,5-dimethoxybenzyl)-N-(5-fluoro-2-phenoxyphenyl)acetamide (hereinafter DAA1106) and N-(2-fluoromethyl-5-methoxybenzyl)-N-(5-fluoro-2-phenoxyphenyl)acetamide (hereinafter FMDAA1106) (Patent Document 2) and N-[2-(2-fluoro)ethyl-5-methoxybenzyl]-N-(5-fluoro-2-phenoxyphenyl)acetamide (hereinafter FEDAA1106) (Patent Document 2) have strong affinity and high selectivity and thus they are suitable for this purpose. That is, [¹¹C] DAA1106 which is labeled with ¹¹C, and [¹⁸F]FMDAA1106 and [¹⁸F] FEDAA1106 which are labeled with ¹⁸F give high signals as PET tracers in the external counting of the intracerebral PBR, which are highly accurate in quantitative analysis.

Patent Document 1: JP 11-171844 A
Patent Document 2: JP 2004-231647 A

### DISCLOSURE OF THE INVENTION

### PROBLEM TO BE SOLVED BY THE INVENTION

The objects of the present invention are to provide compounds which are useful as PBR ligands having strong affinity and high selectivity and to enable the measurement of PBR in a living body by technique including not only PET but also SPECT by labeling the PBR ligands having strong affinity and high selectivity in the external counting of PBR with radioactive halogen nuclides.

### MEANS FOR SOLVING THE PROBLEM

The present inventors have conducted extensive studies for the purpose of solving the above problem, and consequently have found that excellent PBR affinity can be achieved by changing the alkyl group of the compounds described in JP 11-171844 A to a halogenated alkyl group and thus completed the present invention.
That is, the present invention is directed to a radioactive halogen-labeled phenyloxyaniline derivative represented by formula (I) 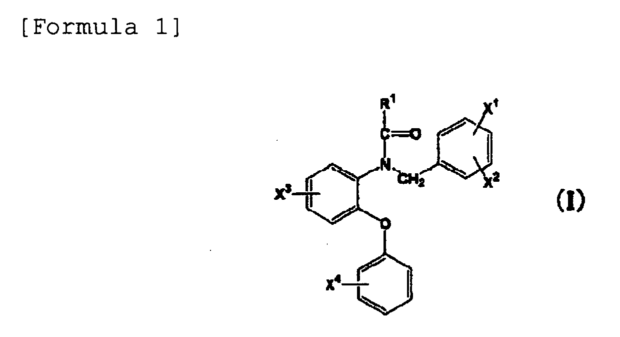 wherein R¹ represents a hydrogen atom, a substituted or unsubstituted alkyl group having 1 to 10 carbon atoms, an alkoxy group having 1 to 10 carbon atoms, a substituted or unsubstituted phenyl group, a group represented by formula -NR²(R³), wherein R² and R³ are the same or different and represent a hydrogen atom, an alkyl group having 1 to 10 carbon atoms, or form a 4-to 10-membered cyclic amino group together with the adjacent nitrogen atom, and X¹, X², X³ and X⁴ are the same or different and each represents a hydrogen atom, an alkyl group having 1 to 5 carbon atoms, an alkoxy group having 1 to 5 carbon atoms, a phenoxy group, a trifluoromethyl group, a carbamoyl group, an aminosulfonyl group, a halogen atom or a radioactive halogen atom selected from the group consisting of ¹²¹I, ¹²³I, ¹²⁴I, ¹²⁵I, ¹³¹I, ⁷⁵Br, ⁷⁶Br, ⁷⁷Br and ^{34m}Cl, provided that at least one of X¹, X², X³ and X⁴ represent a radioactive halogen atom selected from the group consisting of ¹²¹I, ¹²³I, ¹²⁴I, ¹²⁵I, ¹³¹I, ⁷⁵Br, ⁷⁶Br, ⁷⁷Br and ^{34m}Cl.

Preferably, the present invention is directed to a radioactive halogen-labeled phenyloxyaniline derivative according to claim 1, wherein R¹ represents a hydrogen atom, a substituted or unsubstituted alkyl group having 1 to 10 carbon atoms, and X¹, X², X³ and X⁴ are the same or different and each represents a hydrogen atom, an alkoxy group having 1 to 5 carbon atoms, a halogen atom or a radioactive halogen atom selected from the group consisting of ¹²¹I, ¹²³I, ¹²⁴I, ¹²⁵I, ¹³¹I, ⁷⁵Br, ⁷⁶Br, ⁷⁷Br and ^{34m}Cl, provided that at least one of X¹, X², X³ and X⁴ represent a radioactive halogen atom selected from the group consisting of ¹²¹I, ¹²³I, ¹²⁴I, ¹²⁵I, ¹³¹I, ⁷⁵Br, ⁷⁶Br, ⁷⁷Br and ^{34m}Cl.

More preferably, the present invention is directed to a radioactive halogen-labeled phenyloxyaniline derivative according to claim 1, wherein R¹ represents an alkyl group having 1 to 10 carbon atoms, X¹ and X² each represent an alkoxy group having 1 to 5 carbon atoms, X³ represents a radioactive halogen atom selected from the group consisting of ¹²¹I, ¹²³I, ¹²⁴I, ¹²⁵I, ¹³¹I, ⁷⁵Br, ⁷⁶Br, ⁷⁷Br and ^{34m}Cl, and X⁴ represents a hydrogen atom.

Most preferably, the present invention is directed to a radioactive halogen-labeled phenyloxyaniline derivative according to claim 1, wherein R¹ represents an alkyl group having 1 to 10 carbon atoms, X¹ and X² are the same or different and each represents an alkoxy group having 1 to 5 carbon atoms or a radioactive halogen atom selected from the group consisting of ¹²¹I, ¹²³I, ¹²⁴I, ¹²⁵I, ¹³¹I, ⁷⁵Br, ⁷⁶Br, ⁷⁷Br and ^{34m}Cl, provided that either one of X¹ or X² represent a radioactive halogen atom selected from the group consisting of ¹²¹I, ¹²³I, ¹²⁴I, ¹²⁵I, ¹³¹I, ⁷⁵Br, ⁷⁶Br, ⁷⁷Br and ^{34m}Cl, X³ represents a halogen atom, and X⁴ represents a hydrogen atom.

Besides, the present invention is a diagnostic agent of Alzheimer's disease, front-temporal dementia, diffuse Lewy corpuscle disease, vascular lesion, Parkinson's disease-related disease, corticobasilar degeneration, Parkinson's disease, Huntington's chorea, multiple system atrophy, multiple sclerosis, epilepsy, meningitis, encephalitis, peripheral nerve injury, larynx cancer, breast cancer, ovarian tumor, liver cancer, large bowel cancer, stomach cancer, adrenal gland tumor, glioma, glioblastoma, fibroblastoma, neurosarcoma, lung cancer, uterine cancer, lymphoma, prostate cancer, melanoma, testicular tumor, astrocytoma or ectopic hormone-producing tumor, comprising a radioactive halogen-labeled phenyloxyaniline derivative represented by formula (I) as an active ingredient.

### ADVANTAGES OF THE INVENTION

According to the present invention, there have been provided compounds which are useful as PBR ligands having strong affinity and high selectivity.
In addition, measurement of PBR in a living body by technique including not only PET but also SPECT has become possible by labeling the PBR ligands having strong affinity and high selectivity in the external counting of PBR with radioactive halogen nuclides.
This enables early diagnosis of Alzheimer's disease, front-temporal dementia, diffuse Lewy corpuscle disease, vascular disorder, Parkinson's disease-related disease, corticobasilar degeneration, Parkinson's disease, Huntington's chorea, multiple system atrophy, multiple sclerosis, epilepsy, meningitis, encephalitis, peripheral nerve injury, larynx cancer, breast cancer, ovarian tumor, liver cancer, large bowel cancer, stomach cancer, adrenal gland tumor, glioma, glioblastoma, fibroblastoma, neurosarcoma, lung cancer, uterine cancer, lymphoma, prostate cancer, melanoma, testicular tumors, astrocytoma, ectopic hormone-producing tumor, etc. Besides, the compounds of the present invention are useful as prevention and therapeutic drugs of the above diseases.

### BRIEF DESCRIPTION OF THE DRAWINGS

Fig. 1 is an ex vivo autoradiogram of the brain in 30 minutes after [¹³¹I]2IDAA1106 was administered to a rat; and
Fig. 2 is an ex vivo autoradiogram of the brain in 30 minutes after [¹³¹I]2IDAA1106 was administered to a rat.

### BEST MODE FOR CARRYING OUT THE INVENTION

In the present invention, a halogen atom is a fluorine atom, a chlorine atom, a bromine atom or an iodine atom; preferably it is a fluorine atom, an iodine atom or a bromine atom; and more preferably it is a fluorine atom or an iodine atom.

In the present invention, an alkyl group having 1 to 10 carbon atoms refers to a linear, branched or cyclic alkyl group; and, for example, it includes a methyl group, an ethyl group, a propyl group, an isopropyl group, a cyclopropyl group, a butyl group, an isobutyl group, a cyclobutyl group, a cyclopropylmethyl group, a pentyl group, an isopentyl group, a cyclopentyl group, a cyclobutylmethyl group, a 1-ethylpropyl group, a hexyl group, an isohexyl group, a cyclohexyl group, a cyclopentylmethyl group, a 1-ethylbutyl group, a heptyl group, an isoheptyl group, a cyclohexylmethyl group, an octyl group, a nonyl group and a decyl group.

In the present invention, a substituted alkyl group having 1 to 10 carbon atoms refers to an alkyl group substituted with "a hydroxyl group, an alkanoyloxy group, an alkanoyl group, an alkoxy group, a halogen atom, an azido group, an amino group, a carboxyl group"; and, for example, it includes a hydroxymethyl group, an acetyloxymethyl group, methoxymethyl group, a chloromethyl group, a trifluoromethyl group, azidomethyl group, an aminomethyl group, a dimethyl aminomethyl group, a pyrrolidinomethyl group.

In the present invention, an alkoxy group having 1 to 10 carbon atoms refers to a linear, branched or cyclic alkoxy group; and, for example, it includes a methoxy group, an ethoxy group, a propoxy group, an isopropoxy group, a butoxy group, an isobutoxy group, a cyclopropylmethoxy group, a pentyloxy group, an isopentyloxy group, a hexyloxy group, a heptyloxy group, an octyloxy group, a nonyloxy group, a decyloxy group.

In the present invention, a substituted phenyl group refers to a phenyl group substituted with one to three groups selected from the group consisting of an alkyl group having 1 to 10 carbon atoms, an alkyl group having 1 to 10 carbon atoms substituted with "a halogen atom, a hydroxyl group, an alkanoyloxy group having 1 to 10 carbon atoms, a carboxyl group or an alkoxycarbonyl group", an alkenyl group having 2 to 10 carbon atoms, an alkoxy group having 1 to 10 carbon atoms, an alkylthio group having 1 to 10 carbon atoms, a group represented by formula -O-Z-R4 (wherein Z represents a branched or unbranched alkylene group having 1 to 10 carbon atoms and R4 represents an amino group, an amino group substituted with one or two alkyl groups having 1 to 7 carbon atoms, a cyclic amino group having 2 to 7 carbon atoms, a hydroxyl group, a carboxyl group or an alkoxycarbonyl group), an alkanoyl group having 2 to 10 carbon atoms or a ketal thereof, a formyl group or an acetal thereof, a carboxyl group, an alkoxycarbonyl group having 2 to 10 carbon atoms, a carbamoyl group, a carbamoyl group in which the nitrogen atom is substituted with one or two alkyl groups having 1 to 10 carbon atoms, an aminosulfonyl group, an aminosulfonyl group in which the nitrogen atom is substituted with one or two alkyl groups having 1 to 10 carbon atoms, a halogen atom and a nitro group; and it includes, for example, a 2-methylphenyl group, a 2-propylphenyl group, a 2-isopropylphenyl group, a 2-cyclopentylphenyl group, a 2-(1-hydroxyethyl) phenyl group, a 2-carboxymethylphenyl group, a 2-methoxycarbonylphenyl group, a 2-vinylphenyl group, a 2-methoxyphenyl group, a 3-methoxyphenyl group, a 4-methoxyphenyl group, a 2-ethoxyphenyl group, a 2-hexyloxyphenyl group, a 2-isopropoxyphenyl group, a 2-cyclopentoxyphenyl group, a 2,5-dimethoxyphenyl group, a 2,4,6-trimethoxyphenyl group, a 4-methylthiophenyl group, a 2-isopropylthiophenyl group, a 4-cyclohexylthiophenyl group, a 2-(2-dimethylaminoethoxy)phenyl group, a 2-(2-hydroxyethoxy) phenyl group, a 2-carboxymethoxyphenyl group, a 2-methoxycarbonylmethoxyphenyl group, a 2-acetylphenyl group, a 2-(2-methyl-1,3-dioxolan-2-yl)phenyl group, a 2-formylphenyl group, a 2-(1,3-dioxolan-2-yl)phenyl group, a 2-carboxylphenyl group, a 2-(N-methylaminocarbonyl)phenyl group, a 2-(N,N-dimethylaminocarbonyl)phenyl group, a 2-aminocarbonylphenyl group, a 2-aminosulfonylphenyl group, a 4-aminosulfonylphenyl group, a 2-methylaminosulfonylphenyl group, a 2-dimethylaminosulfonylphenyl group, a 2-fluorophenyl group group, a 3-fluorophenyl group, a 4-fluorophenyl group, a 2-chlorophenyl group, a 3-chlorophenyl group, a 4-chlorophenyl group, a 2-bromophenyl group, a 3-bromophenyl group, a 4-bromophenyl group, a 2,4-difluorophenyl group, a 2-nitrophenyl group, a 2-aminophenyl group, a 2-pyrrolidinophenyl group, and a 4-dimethylaminophenyl group.

In the present invention, the 4- to 10-membered cyclic amino group represented by formula - NR2(R3) refers to a cyclic amino group which may contain a nitrogen atom or an oxygen atom; and it includes, for example, a pyrrolidino group, a piperidino group, a piperazino group, a N-methylpiperazino group, a morpholino group.

The compounds of the present invention can be prepared from the compounds prepared in the same method as described in JP 11-171844 A by the method shown below (in the reaction formula, X¹, X² and R¹ mean the same as above.).

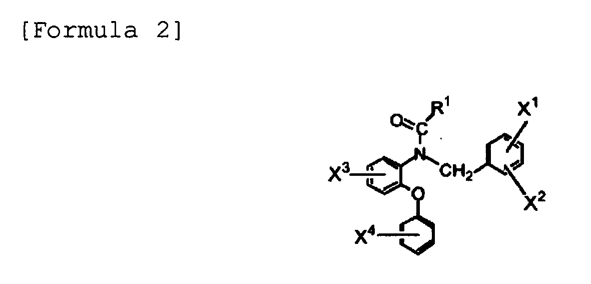

Thus, N-(2-benzyloxy-5-alkoxybenzyl)-N-(phenoxyphenyl)acylamide compounds having various non-radioactive halogen atoms represented by the above formula can be reacted with a palladium complex and an organotin compound to replace the non-radioactive halogen atom with an organotin substituent and then reacted with various radioactive halogen reagents to obtain phenyloxyaniline derivatives labeled with a halogen atom. Here, at least one of X¹, X², X³ and X⁴ represent a radioactive halogen atom selected from the group consisting of ¹²¹I, ¹²³I, ¹²⁴I, ¹²⁵I, ¹³¹I, ⁷⁵Br, ⁷⁶Br, ⁷⁷Br and ^{34m}Cl.

The present invention is described in more detail by way of working examples and test examples.

### Examples

### (Example 1)

### Preparation of [¹³¹I]-N-(2,5-dimethoxybenzyl)-N-(5-iodo-2-phenoxyphenyl)acetamide (hereinafter [¹³¹I]1IDAA1106)

1-1) N-(2,5-dimethoxybenzyl)-N-(5-bromo-2-phenoxyphenyl)acetamide (510 mg, 1.12 mmol) was dissolved in toluene and circulated with hexabutylditin (IV) and dichlorobis(triphenylphosphine)palladium (0) for four days. After the toluene was removed, the reaction product was purified by silica gel column chromatography (eluted by hexane:ethyl acetate = 1:4) to obtain 320 mg (43%) of N-(2,5-dimethoxybenzyl)-N-(5-tributylstannyl-2-phenoxyphenyl)acetamide.
   FABMS C₃₆H₄₈FNO₃Sn (m/z) 680.5 (m⁺+1)
   To a solution of N-(2,5-dimethoxybenzyl)-N-(5-tributylstannyl-2-phenoxyphenyl)acetamide (55 mg, 0.083 mmol) in chloroform was added 100 mg of solid iodine, the resulting reaction liquid was stirred at room temperature for one hour, and then a saturated sodium thiosulfate aqueous solution was add to the reaction liquid till the reaction liquid became colorless. The organic layer was separated, washed with a saturated saline solution and dried over anhydrous magnesium sulfate. The crude product obtained by evaporating the solvent under reduced pressure was purified by silica gel column chromatography (eluted by hexane:ethyl acetate = 1:4) to obtain 32 mg (77%) of the title compound.
1-2) 100 µL of acetic acid /30% hydrogen peroxide (3/1) was added to ethyl acetate ester (100 µL) of N-(2-tributylstannyl-5-methoxybenzyl)-N-(5-fluoro-2-phenoxyphenyl)acetamide (0.1 mg) and mixed. [¹³¹I]NaI (0.1 mCi) was added to the mixture and allowed to stand still for one minute. After completion of the reaction, the reaction mixture was injected into a reversed-phase semi-preparative HPLC (YMC J'sphere ODS-H80 column, 10 mmID × 250 mm). A fraction of [¹³¹I]1IDAA1106 was obtained using CH₃CN/H₂O (9/1) as a mobile phase at a flow rate of 4 mL/min. The solvent was removed under reduced pressure from this fraction and the residue was dissolved in a normal saline solution (1 mL) which was then passed through a 0.22 µm Millipore filter to successfully obtain [¹³¹I]1IDAA1106 (0.091 mCi).

### (Example 2)

### Preparation of N- (2- [¹³¹I]iodo-5-methoxybenzyl)-N-(5-fluoro-2-phenoxyphenyl)acetamide (hereinafter [¹³¹I] 2IDAA1106)

100 µL of acetic acid /30% hydrogen peroxide (3/1) was added to ethyl acetate ester (100 µL) of N-(2-tributylstannyl-5-methoxybenzyl)-N-(5-fluoro-2-phenoxyphenyl)acetamide (0.1 mg) and mixed. [¹³¹I]NaI (0.1 mCi) was added thereto and allowed to stand still for one minute. After completion of the reaction, the reaction mixture was injected into a reversed-phase semi-preparative HPLC (YMC J'sphere ODS-H80 column, 10 mmID x 250 mm). A fraction of [¹³¹I]1IDAA1106 was obtained using CH₃CN/H₂O (9/1) as a mobile phase at a flow rate of 4 mL/min. The solvent was removed under reduced pressure from this fraction and the residue was dissolved in a normal saline solution (1 mL) which was then passed through a 0.22 µm Millipore filter to successfully obtain [¹³¹I]2IDAA1106 (0.095 mCi).

### (Test Example)

### Ex vivo Autoradiography

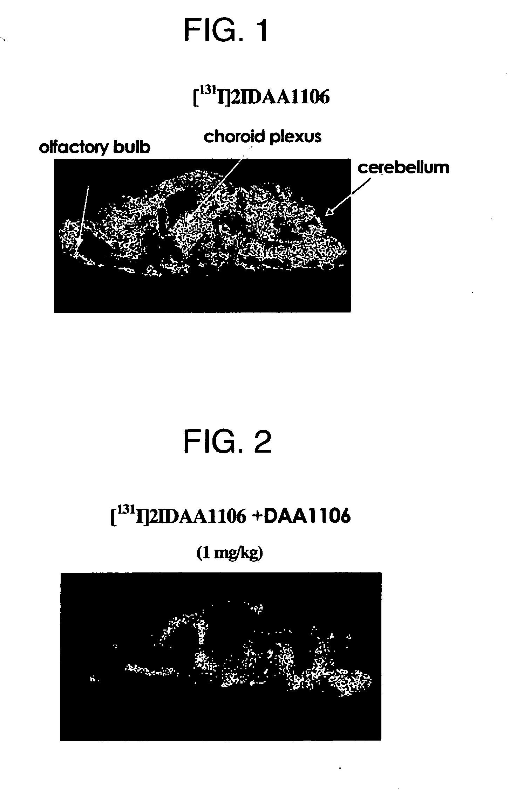

Figs. 1 and 2 show cerebral ex vivo autoradiograms taken in 30 minutes after [¹³¹I]2IDAA1106 was administered to a rat. [¹³¹I]2IDAA1106 showed relatively high brain permeability characteristics as shown in Fig. 1 and met the essential requirements as a radioligand. There was relatively high radioactivity distribution at the olfactory bulb, choroid plexus and cerebellum, and this distribution profile agreed with the intracerebral distribution of peripheral benzodiazepine receptors. In addition, radioactivity distribution decreased in the whole brain as shown in Fig. 2 when DAA1106 and [¹³¹I]2IDAA1106 were administered at the same time. In particular, radioactivity levels in the olfactory bulb, choroid plexus and cerebellum decreased remarkably and were equal to or less than 20% of Fig. 1. These facts demonstrated that [¹³¹I]2IDAA1106 was a radioligand specific to peripheral benzodiazepine receptors. In addition, it was suggested that [¹³¹I]2IDAA1106 could image peripheral benzodiazepine receptors.

In the following, synthesis of the compounds of the present invention having an alkoxy group to which ¹¹C was introduced was shown.

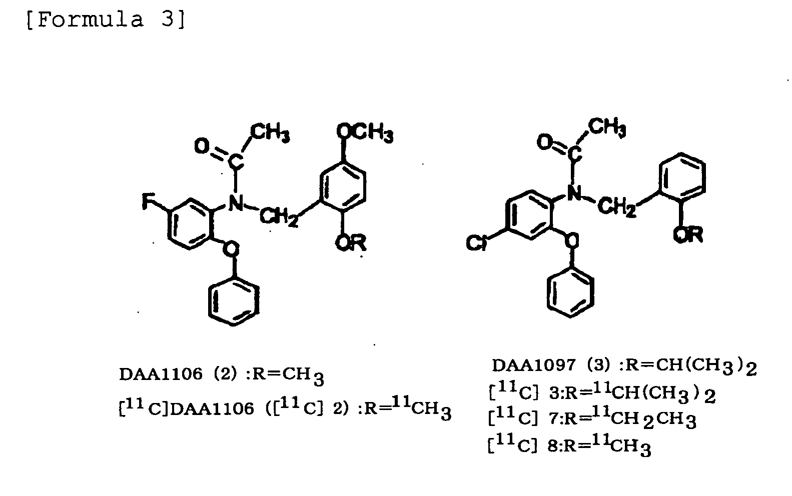

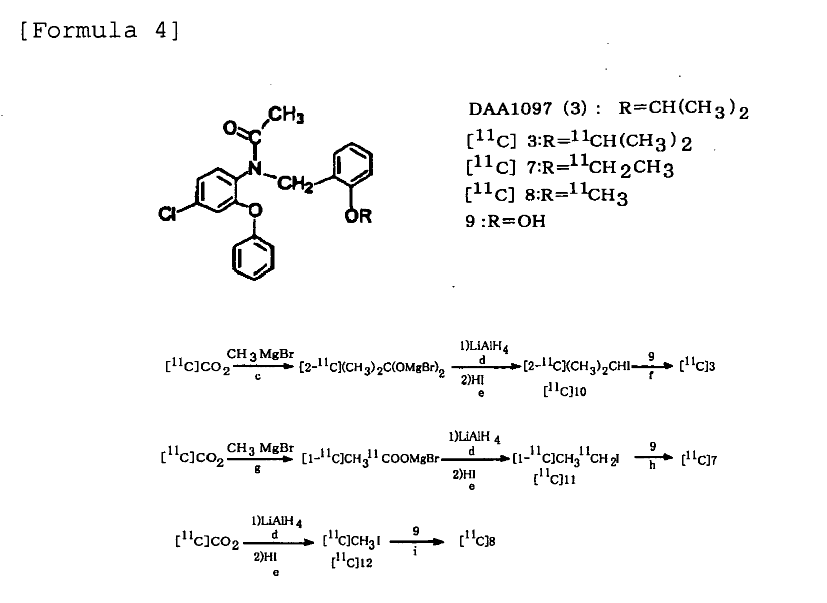

### (Example 3)

### N-(4-chloro-2-phenoxyphenyl)-N-(2-[2-¹¹C] isopropoxybenzyl) acetamide ([¹¹C]3)

A super high purity nitrogen gas (1.5 MPa) containing 0.01% oxygen gas was subjected to irradiation of 18.5 MeV proton from a cyclotron and a carrier-free [¹¹C]CO₂ was produced by ¹⁴N (p, α) ¹¹C nuclear reaction.
After irradiation, [¹¹C]CO₂ was collected from the gas target by N₂ (500 mL/min) and concentrated till the radioactivity in the whole coil reached stable state in a stainless steel coil cooled to -150°C in liquid N₂. The concentrated [¹¹C]CO₂ was discharged when warmed, and the gas was allowed to flow into a loop containing CH₃MgBr with dry N₂ (2 mL/min) at -5°C. When the transfer of radioactivity was completed, N₂ flow was stopped and then this loop was maintained at 25°C for five minutes to perform Grignard reaction. Subsequently, LiA1H₄ solution in THF (0.2 M, 500 µL) was allowed to pass through the loop and the reaction mixture was moved to a heating reactor at 180°C for one minute. After the reactor was cooled at 50 to 60°C, an HI aqueous solution (57%, 800 µL) was added to this reactor. The reaction mixture was heated to 180°C and the generated radioactive fraction was scavenged with N₂ (50 mL /min) and introduced into the inlet of Porapak (trademark) column at ambient temperature. N₂ flow was continued for three minutes till the radioactivity level became stabilized in the column inlet. When this column was heated (heating rate: 15°C /30 second), [¹¹C]10 began to flow from the column outlet in six minutes, which was collected into a pot containing anhydrous DMF (1 mL). Then this [¹¹C]CO₂ was used in the reaction with a Grignard reagent MeMgBr followed by separation by gas chromatography to obtain [¹¹C]10 (3.7 - 4.4 GBq, n = 3) at radiochemical purity of > 95%.
Suspension of 9 (1.0 mg), [¹¹C]10 (3.0 - 3.2 GBq) and NaH in DMF (7 µL, 0.5 g /20 mL DMF) were heated to 130°C and maintained as it was for 10 minutes. The reaction mixture containing [¹¹C]3 was quenched by adding CH₃CN/H₂O (90/10,0.5 mL) and then subjected to a semi-fractionation column (inside diameter 10 mm x 250 mm, CAPCELL PAK C₁₈, SHISEIDO) equipped in a JASCO HPLC system. CH₃CN/H₂O was used on the column for elution at a flow rate of 5.0 mL/min, and the desired fraction (t_{R} =8.8 min) was collected into a flask. After the solvent was vaporized at 90°C under reduced pressure from the flask, the residue was collected in 10 mL of a sterilized saline solution. The saline solution of [¹¹C]3 was passed though a sterilized 0.22 µm filter into a sterilized bottle. At the end of the synthesis, [¹¹C]3 (180 - 310 MBq, n = 3) was obtained at a radiochemical purity of > 98%.

### (Example 4)

### N-(4-chloro-2-phenoxyphenyl)-N-(2-[2-¹¹C] ethoxybenzyl) acetamide ([¹¹C]7)

At the end of the synthesis, [¹¹C]11 (3.9 - 5.3 GBq, n = 3) was obtained at a radiochemical purity of > 95% by performing reaction of CH₃MgBr and [¹¹C]CO₂.
Suspension of 9 (1.0 mg), [¹¹C]11 (3.0 - 3.2 GBq) and NaH (7 µL, 0.5 g /20 mL DMF) in DMF (1 mL) were heated to 50°C and maintained as it was for 5 minutes. The reaction mixture was purified by the same column as used in the above except that CH₃CN/H₂O (80/20) was used, to obtain a desired radioactive fraction (t_{R} =8.1 min). After treated by the same method as performed on [¹¹C]3, [¹¹C]7 (300 - 350 MBq, n = 3) was obtained at a radiochemical purity of > 98% at the end of the synthesis.

### (Example 5)

### N-(4-chloro-2-phenoxyphenyl)-N-(2-[¹¹C]methoxybenzyl)acetamide ([¹¹C]8)

Preparation of [¹¹C]12 and subsequent [¹¹C]methylation of 9 to [¹¹C]8 were performed with an automatic synthesizer. The thus formed [¹¹C]12 was distilled and passed through ascarite and P₂O₅ column, and collected in a container containing 9 (1.0 mg), NaH (7 µL, 0.5 g/20 mL DMF) and DMF (1 mL) for 1.5 minutes at -15 to -20°C. Then the reaction vessel was heated to 30°C and maintained for five minutes. The reaction mixture was purified by the same column as used in the above except that CH₃CN/H₂O (70/30) was used, to obtain a desired radioactive fraction (t_{R} =9.5 min). After treated by the same method as performed on [¹¹C]3, [¹¹C]8 (1.0 - 1.3 GBq, n = 3) was obtained at a radiochemical purity of > 98% at the end of the synthesis.

### (Example 6)

### N-(5-fluoro-2-phenoxyphenyl)-N-(2-[¹¹C]methoxy-5-methoxybenzyl)acetamide ([¹¹C]2)

The title compound was obtained by a method similar to the method as used in Example 5.

### INDUSTRIAL APPLICABILITY

The compounds of the present invention can be used as early-stage diagnostic agents for the diseases such as Alzheimer's disease, front-temporal dementia, diffuse Lewy corpuscle disease, vascular lesion, Parkinson's disease-related disease, corticobasilar degeneration, Parkinson's disease, Huntington's chorea, multiple system atrophy, multiple sclerosis, epilepsy, meningitis, encephalitis, peripheral nerve injury, larynx cancer, breast cancer, ovarian tumor, liver cancer, large bowel cancer, stomach cancer, adrenal gland tumor, glioma, glioblastoma, fibroblastoma, neurosarcoma, lung cancer, uterine cancer, lymphoma, prostate cancer, melanoma, testicular tumors, astrocytoma, and ectopic hormone-producing tumor and also as preventive and therapeutic drugs of the above diseases.

## Claims

1. A radioactive halogen-labeled phenyloxyaniline derivative represented by formula (I) 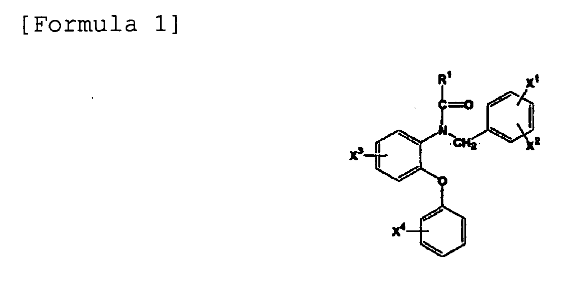 wherein R¹ represents a hydrogen atom, a substituted or unsubstituted alkyl group having 1 to 10 carbon atoms, an alkoxy group having 1 to 10 carbon atoms, a substituted or unsubstituted phenyl group, a group represented by formula -NR²(R³), wherein R² and R³ are the same or different and represent a hydrogen atom, an alkyl group having 1 to 10 carbon atoms, or form a 4-to 10-membered cyclic amino group together with the adjacent nitrogen atom, and X¹, X², X³ and X⁴ are the same or different and each represents a hydrogen atom, an alkyl group having 1 to 5 carbon atoms, an alkoxy group having 1 to 5 carbon atoms, a phenoxy group, a trifluoromethyl group, a carbamoyl group, an aminosulfonyl group, a halogen atom, an alkoxy group having 1 to 5 carbon atoms labeled with ¹¹C or a radioactive halogen atom selected from the group consisting of ¹²¹I, ¹²³I, ¹²⁴I, ¹²⁵I, ¹³¹I, ⁷⁵Br, ⁷⁶Br, ⁷⁷Br and ^{34m}Cl, provided that at least one of X¹, X², X³ and X⁴ represent an alkoxy group having 1 to 5 carbon atoms labeled with ¹¹C or a radioactive halogen atom selected from the group consisting of ¹²¹I, ¹²³I, ¹²⁴I, ¹²⁵I, ¹³¹I, ⁷⁵Br, ⁷⁶Br, ⁷⁷Br and ^{34m}Cl.

2. The radioactive halogen-labeled phenyloxyaniline derivative according to claim 1, wherein R¹ represents a hydrogen atom, a substituted or unsubstituted alkyl group having 1 to 10 carbon atoms, and X¹, X², X³ and X⁴ are the same or different and each represents a hydrogen atom, an alkoxy group having 1 to 5 carbon atoms, a halogen atom, an alkoxy group having 1 to 5 carbon atoms labeled with ¹¹C or a radioactive halogen atom selected from the group consisting of ¹²¹I, ¹²³I, ¹²⁴I, ¹²⁵I, ¹³¹I, ⁷⁵Br, ⁷⁶Br, ⁷⁷Br and ^{34m}Cl, provided that at least one of X¹, X², X³ and X⁴ represent a radioactive halogen atom selected from the group consisting of ¹²¹I, ¹²³I, ¹²⁴I, ¹²⁵I, ¹³¹I, ⁷⁵Br, ⁷⁶Br, ⁷⁷Br and ^{34m}Cl.

3. The radioactive halogen-labeled phenyloxyaniline derivative according to claim 1, wherein
R¹ represents an alkyl group having 1 to 10 carbon atoms,
X¹ and X² each represent an alkoxy group having 1 to 5 carbon atoms,
X³ represents a radioactive halogen atom selected from the group consisting of ¹²¹I, ¹²³I, ¹²⁴I, ¹²⁵I, ¹³¹I, ⁷⁵Br, ⁷⁶Br, ⁷⁷Br and ^{34m}Cl, and
X⁴ represents a hydrogen atom.

4. The radioactive halogen-labeled phenyloxyaniline derivative according to claim 1, wherein
R¹ represents an alkyl group having 1 to 10 carbon atoms,
X¹ and X² are the same or different and each represents an alkoxy group having 1 to 5 carbon atoms or a radioactive halogen atom selected from the group consisting of ¹²¹I, ¹²³I, ¹²⁴I, ¹²⁵I, ¹³¹I, ⁷⁵Br, ⁷⁶Br, ⁷⁷Br and ^{34m}Cl, provided that either one of X¹ or X² represent a radioactive halogen atom selected from the group consisting of ¹²¹I, ¹²³I, ¹²⁴I, ¹²⁵I, ¹³¹I, ⁷⁵Br, ⁷⁶Br, ⁷⁷Br and ^{34m}Cl,
X³ represents a halogen atom, and
X⁴ represents a hydrogen atom.

5. A diagnostic agent of Alzheimer's disease, front-temporal dementia, diffuse Lewy corpuscle disease, vascular lesion, Parkinson's disease-related disease, corticobasilar degeneration, Parkinson's disease, Huntington's chorea, multiple system atrophy, multiple sclerosis, epilepsy, meningitis, encephalitis, peripheral nerve injury, larynx cancer, breast cancer, ovarian tumor, liver cancer, large bowel cancer, stomach cancer, adrenal gland tumor, glioma, glioblastoma, fibroblastoma, neurosarcoma, lung cancer, uterine cancer, lymphoma, prostate cancer, melanoma, testicular tumor, astrocytoma or ectopic hormone-producing tumor, comprising a radioactive halogen-labeled phenyloxyaniline derivative according to any of claims 1 to 4 as an active ingredient.
